# FASCICULE DE BREVET EUROPEEN

(11) **EP 2 931 257 B1**
(45) Date de publication et mention de la délivrance du brevet: **04.09.2019**
(21) Numéro de dépôt: 13818322.3
(22) Date de dépôt: 17.12.2013
(51) Int. Cl.: A61K 9/00, A61K 9/16, A61K 9/20

(54) **COMPRIMES ORODISPERSIBLES OBTENUS PAR COMPRESSION MOULAGE**
DURCH FORMPRESSEN HERGESTELLTE SCHMELZTABLETTEN
ORODISPERSIBLE TABLETS OBTAINED BY COMPRESSION MOULDING

(30) Priorité: 17.12.2012 FR 1262175
(43) Date de publication de la demande: 21.10.2015
(73) Titulaire: ETHYPHARM, 92213 Saint-Cloud Cedex (FR)
(72) Inventeur: DECORTE, Isabelle, F-28150 Beauvilliers (FR); GENDROT, Edouard, F-28500 Garnay (FR); PREVOST, Yann, F-28170 Tremblay Les Villages (FR)
(74) Mandataire: Cabinet Plasseraud
(86) Numéro de dépôt international: PCT/FR2013/053113
(87) Numéro de publication internationale: WO 2014/096669

(56) Documents cités:
- EP-A1- 1 523 974
- EP-A1- 1 681 048
- WO-A1-2009/123626
- WO-A2-2005/105049
- WO-A2-2007/113856
- US-A1- 2009 087 485
- US-A1- 2012 295 916

## Description

La présente invention se rapporte au domaine de la pharmacie, et plus particulièrement à celui de la galénique.

L'invention a pour objet un comprimé orodispersible obtenu par compression moulage (« compression molding »).

Un comprimé orodispersible est une forme solide qui se désintègre ou se dissout dans la bouche, uniquement au contact de la salive, généralement en moins de 60 secondes.

Les comprimés orodispersibles représentent une forme galénique en plein essor, qui s'est beaucoup développée au cours des dernières années. En effet, les comprimés orodispersibles présentent de nombreux avantages et sont particulièrement adaptés aux patients ayant des difficultés de déglutition, par exemple chez les enfants et les personnes âgées. Cependant, ces populations ne sont pas les seules à présenter une dysphagie, puisque environ 30 à 50 % de la population est concernée par ce problème. De nombreux adultes sont également concernés, et notamment les patients présentant des troubles psychiatriques, mais aussi ceux souffrant de troubles thyroïdiens, de la maladie de Parkinson, de maladies de déficience du système immunitaire (SIDA), de reflux gastro-intestinaux, ainsi que les patients souffrant de nausée, vomissement ou mal des transports. Les comprimés orodispersibles sont également adaptés aux personnes n'ayant pas un accès facile à l'eau, notamment lors de voyages. Un autre avantage desdits comprimés est qu'ils permettent une utilisation pratique et discrète.

Pour permettre une désintégration rapide, les comprimés orodispersibles sont de structure poreuse et sont comprimés à des pressions plus faibles que les comprimés conventionnels, les inconvénients étant qu'ils peuvent être plus fragiles et difficiles à manipuler.

Un grand nombre de méthodes pour l'obtention de comprimés orodispersibles a été mis au point durant les dernières années.

Cependant il existe toujours à ce jour certaines caractéristiques limitant le développement industriel des comprimés orodispersibles, notamment leur friabilité trop importante et leur goût et sensation en bouche parfois désagréables.

Ainsi, même si les comprimés orodispersibles restent une forme assez répandue et appréciée des patients, notamment pour leur utilisation pratique et rapide, une étude effectuée par la Demanderesse a montré que le goût et la sensation en bouche d'un comprimé semblent être les paramètres les plus importants pour les patients, et donc le mauvais goût et/ou la sensation désagréable en bouche sont une des causes majeures de la non-observance des traitements médicaux, et donc de leur non-réussite.

L'un des buts de l'invention est donc d'obtenir un comprimé orodispersible présentant une sensation agréable en bouche et/ou un goût agréable en bouche.

Plus précisément, l'un des buts de l'invention est d'obtenir un comprimé orodispersible présentant un temps de désintégration (désagrégation) dans la bouche inférieur à 60 secondes, de préférence inférieur à 40 secondes, et plus préférentiellement encore inférieur à 30 secondes, qui présente une friabilité satisfaisante tout en présentant une sensation agréable et un goût agréable en bouche.

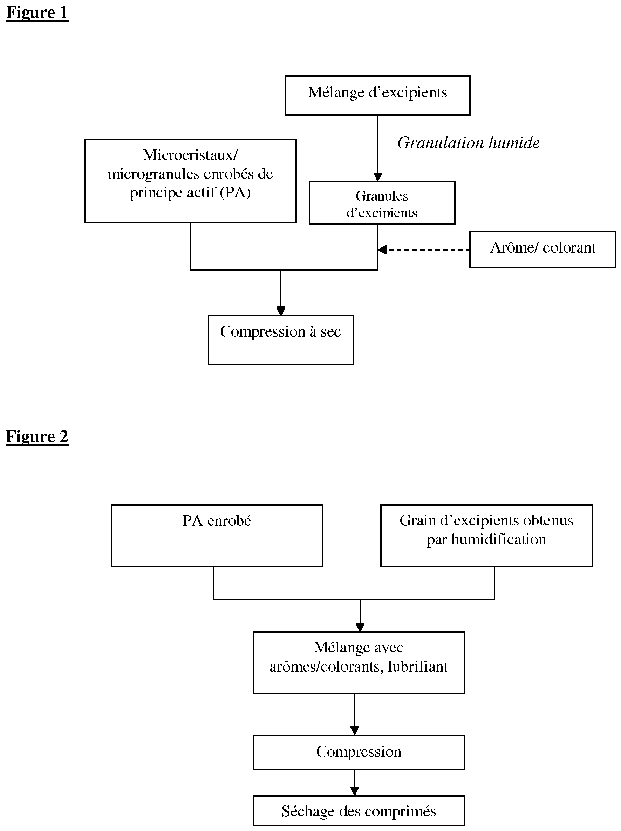

Dans la demande WO 03/039520, les comprimés orodispersibles décrits sont obtenus par une méthode dite de « compression directe », dont les principales étapes sont résumées dans la figure 1. Cependant cette méthode de compression directe n'est pas toujours pleinement satisfaisante, notamment en termes de friabilité et temps de désagrégation des comprimés ainsi obtenus.

Il a maintenant été trouvé de façon surprenante par la Demanderesse que des comprimés orodispersibles aux propriétés très satisfaisantes, telles que décrites ci-dessus, pouvaient être obtenus lorsqu'ils étaient préparés par un procédé de compression moulage, encore appelé « compression molding » par l'homme de l'art.

Le molding (ou moulage en français) est une méthode selon laquelle les comprimés sont formés par compression d'une poudre humide, par solidification d'un gel ou par évaporation d'un solvant. Dans les trois cas, le séchage des comprimés ou évaporation du solvant a lieu après la mise en forme des comprimés. Il existe trois méthodes différentes dites de molding : la technique de « compression molding », de « heat molding » et de « no-vacuum lyophilisation ».

Classiquement, la méthode de « compression molding » est basée sur la compression d'un mélange humide. Cette méthode comprend plusieurs étapes : un mélange de poudres est mouillé par un solvant hydro alcoolique, puis ce mélange humide est comprimé à des forces de compression plus faibles que celles utilisées en compression directe, ce qui entraine une structure très poreuse. La grande porosité des comprimés ainsi obtenus permet une désintégration très rapide (de 5 à 15 secondes). Les comprimés sont ensuite séchés pour en retirer le solvant.

Le procédé de « compression molding » emploie le plus souvent des excipients solubles (saccharides), ce qui contribue également à une désintégration rapide et apporte un goût et une sensation en bouche agréables. D'une formulation à l'autre, la dureté peut être très variable. C'est un paramètre délicat à ajuster, mais il semblerait qu'il soit possible d'obtenir des duretés supérieures à celle obtenues en compression directe.

Dans le brevet US 5,501,861 une méthode de « compression molding » a été développée dans laquelle un mélange humide de principe actif, amidons et sucres est comprimé afin d'obtenir des comprimés poreux, ayant une dureté suffisante pour la fabrication et un temps de désintégration inférieur à 1 minute. Une méthode similaire a également été mise au point dans le brevet US 6,743,443.

Les recherches effectuées par la Demanderesse ont maintenant permis de découvrir que lorsque les comprimés orodispersibles (1) étaient préparés par un procédé de compression moulage et (2) qu'ils présentaient une certaine formulation d'excipients humidifiés, de préférence granulés par humidification, alors lesdits comprimés présentaient des propriétés particulièrement avantageuses, à la fois en terme de dureté, friabilité et sensation en bouche (et bien sûr en temps de désintégration).

Selon un premier objet, la présente invention porte sur un comprimé orodispersible présentant une dureté allant de 30 à 80 N, et de préférence de 40 à 75 N, une friabilité inférieure à 1 % et de préférence inférieure à 0,5 %, une désintégration en bouche inférieure à 60 secondes et de préférence inférieure à 40 secondes, comprenant un principe actif sous forme de microcristaux ou de microgranules enrobés et un mélange d'excipients choisis dans le groupe comprenant un diluant, un désintégrant, un édulcorant, un liant, un agent d'écoulement, un humectant ou agent mouillant, un lubrifiant, un agent aromatisant, un colorant et leurs mélanges, ledit mélange d'excipients se présentant de préférence sous forme de grains, caractérisé en ce que ledit comprimé est obtenu par un procédé de compression moulage (« compression molding ») comprenant les étapes suivantes :
- préparation par humidification du mélange d'excipients, de préférence sous forme de grains, présentant une humidité résiduelle ou teneur en eau allant de 0,5 % à 7 %, de préférence de 1 à 5 %, et plus préférentiellement de 2 à 4%,
- préparation de microcristaux ou de microgranules de principe actif enrobés,
- mélange des microcristaux ou microgranules de principe actif enrobés et du mélange humide d'excipients tel que préparé ci-dessus, ledit mélange d'excipients étant de préférence sous forme de grains,
- éventuellement, ajout au mélange humide pour compression, tel que préparé ci-dessus, d'excipients choisis dans le groupe comprenant un agent d'écoulement, un lubrifiant, un aromatisant, un édulcorant, un colorant et leurs mélanges,
- compression du mélange pour compression préparé ci-dessus pour obtenir un comprimé,
- éventuellement séchage du comprimé ainsi obtenu.

Le mélange humide d'excipients peut se trouver soit sous forme d'une poudre humide, soit sous forme de grains humides.

Les termes « mélange humide d'excipients » ou « mélange d'excipients humides » ont la même signification et pourront être utilisés indifféremment dans ce qui suit.

Lorsque le mélange humide d'excipients est sous forme de grains humides, les propriétés de compression du mélange lors de la préparation du comprimé sont améliorées. Ainsi selon l'invention, le mélange humide d'excipients se présente de préférence sous forme de grains humides d'excipients.

Par « humidification », on entend une étape de mouillage du mélange d'excipients. Cette première étape du procédé se distingue d'une granulation humide laquelle comprend l'utilisation d'une quantité de solvant beaucoup plus importante de façon à mouiller le mélange et à augmenter la granulométrie du mélange. Lors de la granulation humide, une étape de séchage des grains est également nécessaire avant de comprimer le mélange.

Au sens de la présente invention, un comprimé orodispersible est un comprimé qui se désintègre ou se dissout dans la bouche, uniquement au contact de la salive, sans apport d'eau et sans être mâché, en moins de 60 secondes, de préférence en moins de 40 secondes, et plus préférentiellement encore en moins de 30 secondes, en formant une suspension facile à avaler.

Le temps de désintégration (ou désagrégation) dans la bouche correspond à la durée qui sépare, d'une part, le moment de la mise en place du comprimé dans la bouche au contact de la salive et, d'autre part, le moment de la déglutition de la suspension résultant de la désintégration (désagrégation) du comprimé au contact de la salive. Ce temps de désintégration correspond au temps de désintégration *in vivo.*

On peut également mesurer le temps de désintégration *in vitro* des comprimés orodispersibles selon l'invention. Ce temps de désintégration est mesuré selon la Pharmacopée Européenne 2.9.1 sur un appareil Erweka ZT 31 ou tout autre appareil de mesure du temps de désintégration des comprimés, correspondant à la Pharmacopée Européenne 2.9.1. Le temps de désintégration *in vitro* des comprimés selon l'invention est de 10 à 20 secondes.

Les comprimés obtenus par compression moulage sont aussi durs que ceux obtenus par compression directe, mais ils présentent l'avantage de se désintégrer plus rapidement que ceux obtenus par compression directe.

Selon un mode de réalisation avantageux de l'invention, le mélange d'excipients comprend au moins un humectant.

Selon l'invention, les termes « humectant » ou « agent humectant » ont la même signification que « agent mouillant » et pourront donc être utilisés indifféremment dans ce qui suit.

Selon un mode de réalisation avantageux de l'invention, l'humectant est choisi dans le groupe comprenant les poloxamers, de préférence le « poloxamer 188 » ou le « poloxamer 407 », les macrogols, les macrogolglycérides, les polysorbates, ledit humectant étant de préférence un macrogolglycéride tel que le stéaroyl macrogol-32 glycéride ou le lauroyl macrogol-32 glycéride commercialisé sous la dénomination Gelucire ® 44/14.

Selon un mode de réalisation avantageux de l'invention, le mélange d'excipients, de préférence sous forme de grains, comprend :
- de 65 à 90 %, et de préférence de 70 à 80 %, d'un diluant choisi dans le groupe comprenant le mannitol, le xylitol, le sorbitol, le maltitol et leurs mélanges, ledit diluant étant de préférence le mannitol commercialisé sous la dénomination Mannitol 60,
- de 2 à 25 %, et de préférence de 10 à 20 %, d'un désintégrant choisi dans le groupe comprenant la crospovidone, la croscarmellose sodique (AcDiSol®), le carboxyméthylamidon sodique (Explotab ®) et leurs mélanges, ledit désintégrant étant de préférence la crospovidone commercialisée sous la dénomination Polyplasdone ® XL,
- de 1 à 8 %, et de préférence de 3 à 5 %, d'un édulcorant choisi dans le groupe comprenant l'aspartam, l'acesulfame de potassium, la saccharinate de sodium, le sucralose et leurs mélanges, ledit édulcorant étant de préférence l'aspartam,
- de 3 à 10 %, et de préférence de 5 à 8 %, d'un liant choisi dans le groupe comprenant l'hydroxypropylcellulose faiblement substituée, la gomme arabique, l'amidon de maïs, l'amidon prégélatinisé, les maltodextrines et leurs mélanges, ledit liant étant de préférence la gomme arabique et/ou l'hydroxypropylcellulose commercialisée sous la dénomination L-HPC LH 21,
- de 0 à 5 %, et de préférence de 1 à 3 %, d'un agent d'écoulement choisi dans le groupe comprenant de la silice, de préférence celle commercialisée sous la dénomination Syloid ® 244 FP, de la silice colloïdale hydrophobe, de préférence celle commercialisée sous la dénomination Aerosil ® R 972, de la silice précipitée, de préférence celle commercialisée sous la dénomination Aerosil ® 200, et leurs mélanges,
- de 0 à 5 %, et de préférence de 0,1 à 3 %, d'un humectant, ledit humectant étant tel que défini précédemment,
- de 0 % à 5 % d'un lubrifiant, ledit lubrifiant étant de préférence un lubrifiant hydrophile choisi dans le groupe comprenant le stéaryl fumarate de sodium, le lauryl sulfate de sodium, ledit lubrifiant hydrophile étant de préférence le stéaryl fumarate de sodium commercialisé par exemple sous la dénomination Pruv ®,
- de 0 à 8 %, et de préférence de 0,5 à 4 %, d'un agent aromatisant et/ou d'un colorant,
- de l'eau qsp 100 %,
les pourcentages étant des pourcentages en poids par rapport au poids total du mélange d'excipients, ledit mélange d'excipients se présentant de préférence sous forme de grains.

L'agent aromatisant et le colorant pouvant entrer dans la constitution du mélange d'excipients, de préférence sous forme de grains, sont choisis parmi ceux qui sont pharmaceutiquement acceptables. Ils sont choisis selon les caractéristiques organoleptiques souhaitées pour le comprimé orodispersible et de façon à masquer au mieux le goût résiduel du principe actif.

Selon un mode de réalisation particulier de l'invention, la préparation du mélange humide d'excipients, de préférence sous forme de grains, est effectuée par humification du mélange d'excipients, de préférence sous forme de grains, à l'aide d'une solution d'humidification choisie dans le groupe comprenant de l'eau, une solution aqueuse d'humidification, un humectant ou agent mouillant, une solution alcoolique et leurs mélanges, ladite solution d'humidification étant incorporée dans le mélange d'excipients, de préférence sous forme de grains, en une quantité permettant d'obtenir un mélange humide d'excipients, de préférence sous forme de grains, présentant une humidité résiduelle allant de 0,5 % à 7 %, de préférence de 1 à 5 %, et plus préférentiellement de 2 à 4%.

Ainsi selon l'invention, un mélange humide d'excipients désigne un mélange d'excipients présentant une humidité résiduelle allant de 0,5 à 7 %, de préférence de 1 à 5 %, et plus préférentiellement de 2 à 4%.

Dans ce qui précède et dans ce qui suit, lorsque le mélange d'excipients se trouve sous forme de grains, alors le mélange humide d'excipients désigne bien évidemment des grains humides d'excipients.

L'humidité résiduelle est mesurée soit par la méthode de Karl Fisher, soit par une balance à dessiccation (15 minutes à 80 °C).

De façon avantageuse, la solution d'humidification comprend une solution aqueuse d'humidification et un humectant, ledit humectant étant de préférence le lauroyl macrogol-32 glycéride commercialisé sous la dénomination Gelucire ® 44/14.

Selon un autre mode de réalisation avantageux, la solution d'humidification comprend de l'eau et un humectant tel que défini ci-dessus.

Selon encore un autre mode de réalisation avantageux, l'eau est utilisée comme solution d'humidification pour la granulation par humidification.

Selon un mode de réalisation particulier de l'invention, certains excipients du comprimé orodispersible ne se trouvent pas dans le mélange humide d'excipients, de préférence sous forme de grains, mais sont ajoutés en plus au mélange humide d'excipients, de préférence sous forme de grains, dans le mélange pour compression. Ces excipients sont choisis dans le groupe comprenant :
- de 1 à 5 %, de préférence de 2 à 4 %, d'un agent d'écoulement,
- de 1 % à 5 %, de préférence de 2 à 4 %, d'un lubrifiant,
- de 0 à 5 %, et de préférence de 0,5 à 4 %, d'un agent aromatisant et/ou d'un colorant, lesdits agents d'écoulement et lubrifiant étant tels que définis précédemment,
les pourcentages étant des pourcentages en poids par rapport au poids total du mélange pour compression.

Ainsi, certains excipients tels que l'agent d'écoulement, le lubrifiant, l'agent aromatisant et/ou le colorant, peuvent être introduits :
- lors de la préparation du mélange humide d'excipients, par exemple lors de la fabrication du grain (granulé) humide d'excipient et/ou,
- après la fabrication du grain humide d'excipient, par mélange lors de la préparation du mélange pour compression.

Selon un mode de réalisation avantageux de l'invention, le mélange pour compression comprend :
- de 5 à 40 %, de préférence de 10 à 30 %, et plus préférentiellement encore de 15 à 25 % de microcristaux ou de microgranules de principe actif enrobés,
- de 55 à 95 %, de préférence de 65 à 85 %, et plus préférentiellement encore de 70 à 80 % du mélange humide d'excipients, de préférence sous forme de grains,
- de 0 à 10 %, de préférence de 1 à 7 %, et plus préférentiellement encore de 2 à 5 % d'excipients ne se trouvant pas dans le mélange humide d'excipients, de préférence sous forme de grains,
les pourcentages étant des pourcentages en poids par rapport au poids total du mélange pour compression.

Selon l'invention, le mélange pour compression présente une humidité résiduelle ou teneur en eau allant de 0,1 % à 6 %, et de préférence de 2 à 3 %.

Selon un autre mode de réalisation avantageux de l'invention, le mélange pour compression est comprimé à l'aide de forces de compression allant de 8 à 22 kN (kNewtons), de préférence de 10 à 20 kN, et plus préférentiellement encore de 12 kN à 18 kN.

Selon un autre mode de réalisation avantageux, le séchage du comprimé obtenu à l'issue de l'étape de compression, est effectué à une température allant de 35 à 65 °C, de préférence de 45 à 55 °C, pendant un temps allant de 30 min à 3 heures, et de préférence de 1 à 2 heures.

A titre indicatif, le comprimé orodispersible de l'invention avant séchage présente une dureté allant de 45 à 80 N, et de préférence de 50 à 75 N, tandis qu'après séchage il présente une dureté allant de 30 à 70 N, et de préférence de 30 à 65 N.

La méthode de mesure de la dureté provient de la Pharmacopée Européenne 2.9.8.

A titre indicatif, le comprimé orodispersible de l'invention avant séchage présente une friabilité inférieure à 0,8%, et de préférence allant de 0,2 à 0,7%, tandis qu'après séchage il présente une friabilité inférieure à 0,4%, et de préférence allant de 0,1 à 0,3%.

La friabilité est mesurée est mesurée sur un appareil Erweka TA 10 selon la méthode décrite à la Pharmacopée Européenne (édition 7, chapitre 2.9.7.) En raison de cette friabilité satisfaisante, il est possible d'utiliser des méthodes industrielles classiques de transfert et de conditionnement des comprimés ne nécessitant pas de précautions particulières et permettant une grande rapidité d'exécution.

La présente invention a encore pour objet un procédé de préparation d'un comprimé orodispersible comprenant les étapes de:
- préparation par humidification d'un mélange d'excipients, ledit mélange d'excipients se trouvant de préférence sous forme de grains, ledit mélange d'excipients étant choisi dans le groupe comprenant un diluant, un désintégrant, un édulcorant, un liant, un agent d'écoulement, un humectant ou agent mouillant, un lubrifiant, un agent aromatisant, un colorant et leurs mélanges, ledit mélange d'excipients présentant une humidité résiduelle ou teneur en eau allant de 0,5 % à 7 %, de préférence de 1 à 5 %, et plus préférentiellement de 2 à 4%,
- préparation de microcristaux ou de microgranules de principe actif enrobés,
- mélange des microcristaux ou microgranules de principe actif enrobés d'une part et du mélange humide d'excipients d'autre part, ledit mélange humide d'excipients se trouvant de préférence sous forme de grains humides d'excipients,
- éventuellement, ajout au mélange humide pour compression, tel que préparé ci-dessus, d'excipients choisis dans le groupe comprenant un agent d'écoulement, un lubrifiant, un arôme, un édulcorant et leurs mélanges,
- compression du mélange pour compression préparé ci-dessus pour obtenir un comprimé,
- éventuellement séchage du comprimé ainsi obtenu.

Lesdits excipients utilisés dans le procédé de préparation de l'invention, et en particulier lesdits diluant, désintégrant, édulcorant, liant, agent d'écoulement, humectant, lubrifiant, agent aromatisant et colorant sont tels que définis précédemment quant à leur nature et/ou quantité.

Selon un mode de réalisation avantageux du procédé de l'invention, le mélange d'excipients comprend au moins un humectant choisi dans le groupe comprenant les poloxamers, de préférence le « poloxamer 188 » ou le « poloxamer 407 », les macrogols, les macrogolglycérides, les polysorbates, ledit humectant étant de préférence un macrogolglycéride tel que le stéaroyl macrogol-32 glycéride ou le lauroyl macrogol-32 glycéride commercialisé sous la dénomination Gelucire ® 44/14.

Selon un autre mode de réalisation avantageux du procédé de l'invention, la préparation du mélange humide d'excipients, de préférence sous forme de grains, est effectuée par humification du mélange d'excipients à l'aide d'une solution d'humidification choisie dans le groupe comprenant de l'eau, une solution aqueuse d'humidification, un humectant, une solution alcoolique et leurs mélanges, ladite solution d'humidification étant incorporée dans le mélange d'excipients en une quantité permettant d'obtenir un mélange d'excipients présentant une humidité résiduelle telle que définie ci-dessus, ledit mélange humide d'excipients étant de préférence sous forme de grains humides.

L'invention porte également sur un comprimé orodispersible susceptible d'être obtenu selon le procédé tel que décrit ci-dessus, caractérisé en ce qu'il présente :
- une dureté allant de 30 N à 80 N, de préférence de 40 à 75 N et/ou,
- une friabilité inférieure à 1%, et de préférence inférieure à 0,5 %.

Selon un mode de réalisation particulier de l'invention, le comprimé orodispersible tel que défini ci-dessus présente en outre :
- pas ou peu d'effet noyau en bouche, et/ou
- une texture agréable en bouche et/ou un goût agréable en bouche.

Au sens de la présente invention, « l'effet noyau » caractérise une agglomération plus importante des particules au centre du comprimé, qui reste dur plus longtemps et persiste en bouche alors que les couches extérieures du comprimé se désagrègent plus rapidement.

Ainsi s'il existe un effet noyau, le temps de désagrégation en bouche est allongé. De manière préférée, l'effet noyau doit être le plus faible possible.

Au sens de l'invention, une sensation agréable en bouche désigne une texture agréable en bouche, c'est-à-dire l'absence de sensation pâteuse ou granuleuse, et/ou un goût agréable en bouche.

A titre d'exemple, le comprimé orodispersible de l'invention présente :
- une masse allant de 200 à 600 mg, et de préférence de 300 à 500 mg,
- une épaisseur allant de 1 à 5 mm, et de préférence de 2 à 4 mm
- un diamètre de 8 à 14 mm, et de préférence de 9 à 12 mm.

Ainsi de tels comprimés sont d'une taille trop importante pour être avalés, mais peuvent facilement être placés dans la cavité buccale, sur la langue où ils se désintègrent du fait de la présence de la salive et de la pression naturelle exercée entre la langue et le palais lorsque la bouche est refermée.

Selon un autre mode de réalisation, les comprimés peuvent comprendre au moins une encoche permettant leur rupture afin d'administrer une quantité moindre de principe actif.

Le comprimé orodispersible de l'invention est adapté pour la mise en oeuvre de tout type de principe actif se présentant sous forme de microcristaux ou pouvant être granulé.

A titre d'exemple, le principe actif pourra être choisi dans le groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Le comprimé orodispersible de l'invention est particulièrement adapté aux principes actifs utiles dans les traitements destinés aux enfants ou aux personnes âgées compte tenu de leur facilité de prise.

Le principe actif est présent dans le comprimé orodispersible sous forme de microcristaux ou microgranules enrobés.

Les particules de principes actifs présentent une taille allant de 10 à 500 µm.

L'enrobage des microcristaux ou microgranules de principe actif peut être effectué selon l'une des méthodes décrites dans les demandes de brevet FR9109245, FR9704234, FR9806384 et FR0014803.

La composition de la couche fonctionnelle d'enrobage est choisie en fonction des caractéristiques de masquage de goût et/ou de libération de principe actifs souhaitées.

Selon un mode de réalisation préféré de lorsque le mélange d'excipients est sous forme de grains, lesdits grains présentent une granulométrie médiane l'invention, comprise entre + 30 % et - 30 %, de préférence entre + 10 % et - 10%, par rapport à la dimension des microcristaux ou microgranules enrobés.

Selon un mode de réalisation avantageux le comprimé orodispersible de l'invention est tel que la dimension moyenne des microcristaux ou microgranules de principe actif enrobés est de 100 µm à 500 µm, de préférence de 200 µm à 400 µm et la dimension des grains d'excipients est de 70 µm à 650 µm, de préférence de 180 µm à 440 µm.

L'invention sera mieux comprise à la lumière des exemples non limitatifs et purement illustratifs suivants et des figures.

Dans ce qui suit les abréviations suivantes seront utilisées :
PA : Principe Actif
ODT : "Orally Disintegrating Tablet", à savoir « comprimé orodispersible »
FT ou ODT FT : comprimé orodispersible Flashatb ® de référence (obtenu par compression directe)
CD ou ODT CD : comprimé orodispersible obtenu par compression directe
Hum ou ODT Hum : comprimé orodispersible obtenu selon le procédé de l'invention
HR : Humidité résiduelle, teneur en eau
KF : Karl Fischer
LOD : « Loss on Drying », à savoir « perte à la dessiccation »
m/m : masse/masse
Moy. : moyenne
NE : non effectué

Les figures 1 à 5 permettent d'une part d'illustrer une méthode de l'art antérieur de préparation des comprimés orodispersibles, et d'autre part d'illustrer les exemples ci-dessous.

La figure 1 représente les principales étapes de la méthode de compression directe utilisée dans la demande WO 03/039520 déposée au nom de la demanderesse

La figure 2 représente la méthode de « compression molding » selon l'invention.

Les figures 3 et 4 représentent respectivement la friabilité et le temps de désagrégation *in vitro* des comprimés orodispersibles « FT », « CD » et « Hum » ayant une dureté de 50 N (fig. 3) et 70 N (fig. 4).

La figure 5 représente les résultats des essais *in vivo* (effet noyau, sensation en bouche et temps de désagrégation *in vivo*) des comprimés orodispersibles « FT », « CD » et « Hum » ayant une dureté de 50 N.

### EXEMPLES

Tous les ODT testés ci-après sont des placebos. Cependant, pour mimer le principe actif, 20 % de « Neutres », à savoir un « mime » des granules enrobés de principe actif, présentant un diamètre allant de 500 à 600 µm de diamètre, ont été incorporés dans les ODT.

En effet, tous les principes actifs utilisés par la Demanderesse dans les ODT sont enrobés afin de masquer leur goût, ce qui explique une taille des particules assez importante. Le grade particulier de Neutres a été choisi pour des raisons de disponibilités industrielles.

Des comprimés orodispersibles tels qu'obtenus dans la demande WO 03/039520, par compression directe, sont indifféremment représentés dans ce qui suit par « Flashtab ® », FT ou ODT FT, et sont préparés afin de pouvoir les comparer aux ODT de l'invention (représentés dans ce qui suit par Hum ou ODT Hum).

### Exemple 1 (Comparatif): Préparation des « ODT FT » par compression directe

### Matériels et méthodes

Les matières premières utilisées dans les ODT FT sont indiquées dans le tableau 1.

**Tableau 1 :**

| **Noms** | **Fabricant** | **Fonction** |
|---|---|---|
| Neutres 500/600 | NP Pharm | Mime le PA |
| Pearlitol® SD 200 et 160C | Roquette | Diluant |
| Polyplasdone® XL | ISP | Désintégrant |
| Aspartam | Ajimoto | Edulcorant |
| Syloid® 244 FP | Grace Davison | Agent d'écoulement |
| Stéarate de magnésium | Peter Greven | Lubrifiant |

La préparation des mélanges se fait dans un Lodige type FM 50 E (mélangeur granulateur à socs) puis dans des mélangeurs cubiques Frogerais 27 ou 60 L.

Le séchage se fait dans une étuve Binder APT.line™ FP équipée de plateaux perforés.

Le mélange est comprimé sur une presse rotative Fette P1200 équipée d'une assistance mécanique à l'alimentation.

La masse, l'épaisseur et la dureté des comprimés sont contrôlées sur un Checkmaster 4 Fette.

La friabilité est mesurée sur un appareil Erweka TA 10 selon la méthode décrite à la Pharmacopée Européenne (édition 7, chapitre 2.9.7.)

Les tests de désagrégation *in vitro* sont réalisés sur 6 comprimés ODT FT sur un appareil Erweka ZT 31.

La mesure de l'humidité résiduelle est mesurée sur un Karl Fischer Mettler Toledo DL 31, ainsi que sur un Moisture Analyser Mettler Toledo HR 83 et Sartorius MA 100.

Les mesures de densité sont effectuées sur un appareil Tap density Vankel selon la méthode décrite à la Pharmacopée Européenne (édition 7.0, chapitre 2.9.34.) L'indice de Carr est également calculé selon la Pharmacopée Européenne.

Le tableau 2 ci-dessous représente la composition du mélange pour compression des ODT FT.

**Tableau 2 : ODT FT**

| **Noms** | **Pourcentages** |
|---|---|
| Neutres 500/600 | 20,0 |
| Pearlitol® SD 200 | 42,0 |
| Pearlitol® 160C | 26,5 |
| Polyplasdone® XL | 7,0 |
| Aspartam | 2,0 |
| Syloid® 244 FP | 1,0 |
| Stéarate de magnésium | 1,5 |
| **TOTAL** | 100,0 |

Les ODT FT de référence sont fabriqués par compression directe. Le mélange est comprimé sur une presse rotative FETTE P1200, avec des poinçons de type rond, plat, chanfrein de 12 mm de diamètre. Les valeurs cibles de dureté sont 50 et 70 N.

On mesure la masse, l'épaisseur, la dureté, la friabilité et le temps de désintégration des comprimés obtenus. Les mesures de masse, d'épaisseur et de dureté sont effectuées sur 10 comprimés de 12 mm de diamètre.

Des essais *in vivo* ont été réalisés sur les ODT FT. Ces tests ont été réalisés par 11 adultes volontaires. Chaque volontaire testait en moyenne 4 à 6 comprimés, avec la possibilité de boire entre chaque prise si besoin. Chaque comprimé a été testé par 3 personnes.

Les paramètres évalués sont :
- le temps de désagrégation en bouche *in vivo* (chronométré),
- la présence ou non d'un effet noyau,
- la sensation en bouche.

### Résultats et discussion

Le tableau 3 ci-dessous indique les caractéristiques des ODT FT (dont la composition est indiquée dans le tableau 2), en fonction des forces de compression exercées.

**Tableau 3 : ODT FT**

| **Dureté (N)** | **Force de compression (kN)** | **Séchage (h)** | **Masse (mg)** | **Epaisseur (mm)** | **Friabilité (%)** | **Désag. *in vitro* (s)** | | **Ecoulement du mélange** |
|---|---|---|---|---|---|---|---|---|
| | | | | | | **min** | **max** | |
| 46 | 11,0 | 0 | 447 | 3,46 | 0,41 | 12,7 | 17,7 | Bon |
| 73 | 15,7 | 0 | 445 | 3,33 | 0,15 | 16,2 | 21,9 | |

Le tableau 4 ci-dessous représente les essais *in vivo* pour lesdits ODT FT (obtenus par compression directe).

**Tableau 4**

| **Désag. en bouche (s)** | **Effet noyau** | **Sensation en bouche** |
|---|---|---|
| 33 | important | Désagrégation longue à démarrer |

En bouche, les comprimés présentent un effet noyau important, une sensation assez agréable mais une désagrégation longue à démarrer, bien que le temps de désintégration soit satisfaisant.

### Exemple 2

Les matières premières utilisées sont indiquées dans le tableau 5 ci-dessous.

**Tableau 5**

| **Noms** | **Fabricant** | **Fonction** |
|---|---|---|
| Neutres 500/600 | NP Pharm | mime le PA |
| Mannitol 60 | Roquette | Diluant |
| Polyplasdone® XL | ISP | Désintégrant |
| Aspartam | Ajimoto | Edulcorant |
| Syloid® 244 FP | Grace Davison | Agent d'écoulement |
| Aerosil® R 972 | Evonik | Agent d'écoulement |
| L-HPC LH 21 | Seppic | Liant et désintégrant |
| Pruv® | JRS Pharma | Lubrifiant hydrophile |
| Aerosil® 200 | Evonik | Agent d'écoulement |
| Gomme arabique | Carlo Erba | Liant |
| Gelucire® 44/14 | Gattefossé | Humectant |
| AcDiSol® | FMC Biopolymer | Désintégrant |
| Explotab® | JRS Pharma | Désintégrant |

Les appareils utilisés sont les mêmes que ceux décrits dans l'exemple 1 ci-dessus.

Le mélange sous forme de grains est préparé dans le Lodige, puis les excipients pour compression sont ajoutés en phase externe (Syloid®, Aerosil®, Pruv®) avec les Neutres 500/600. Ce mélange est comprimé en utilisant des poinçons (type rond, plat, chanfrein) de 12 mm.

Les valeurs cibles de dureté 50 et 70 N sont visées pour chaque essai.

Pour chaque type de comprimé obtenu, la masse, l'épaisseur, la dureté, la friabilité et le temps de désintégration sont mesurés. Les mesures de masse, épaisseur et dureté sont effectuées sur 10 comprimés.

Pour l'essai en compression directe, les tests de friabilités et désagrégation n'ont été réalisés qu'une seule fois en raison de la faible taille des lots.

Pour tous les essais suivants avec humidification, les tests ont été réalisés 3 fois à partir d'un même lot. Les comprimés sont conservés dans des flacons en verre afin de les protéger de l'humidité.

Pour les tests *in vivo,* la méthode est la même que celle décrite à l'exemple 1.

A l'aide de ces matières premières, ont été préparés des comprimés par compression directe ODT CD (à titre de comparaison) et des comprimés selon l'invention par compression moulage.

### Préparation des ODT CD (comparatif)

Le tableau 6 ci-dessous indique la composition centésimale du mélange pour compression permettant d'obtenir un ODT CD, l'humidité relative de ce mélange est donnée dans le tableau 7.

**Tableau 6 : ODT CD**

| **Noms** | **Pourcentages** |
|---|---|
| Neutres 500/600 | 20,00 |
| Mannitol 60 | 53,00 |
| Polyplasdone XL | 13,40 |
| Aspartam | 4,50 |
| L-HPC LH 21 | 4,70 |
| Syloid® FP 244 | 0,90 |
| Aerosil® R 972 | 2,00 |
| Pruv® | 1,50 |
| **Total** | 100,00 |

**Tableau 7**

| | **LOD (%)** | **KF (%)** |
|---|---|---|
| Mélange pour compression | 1,4 | 1,5 |

La compression est réalisée sur le même type d'appareillage que dans l'exemple 1, à deux forces de compression distinctes pour viser une dureté de 50N ou 70N.

Les mêmes tests que dans l'exemple 1 ont été réalisés.

Le tableau 8 ci-dessous indique les caractéristiques des ODT CD (obtenus par compression directe).

**Tableau 8**

| **Dureté (N)** | **Force de compression (kN)** | **Séchage (h)** | **Masse (mg)** | **Epaisseur (mm)** | **Friabilité (%)** | **Désag. *in vitro* (s)** | |
|---|---|---|---|---|---|---|---|
| | | | | | | **min** | **max** |
| 46 | 20,0 | 0 | 457 | 3,41 | 0,50 | 21 | 24 |

Les résultats des essais *in vivo* sont donnés dans le tableau 9.

**Tableau 9 : ODT CD**

| **Désag. en bouche (s)** | **Effet noyau** | **Sensation en bouche** |
|---|---|---|
| 30 | important | Désagrégation longue, un peu pâteux |

### Préparation de comprimés selon l'invention (méthode de compression moulage)

Une formule similaire à celle de l'essai en compression directe est reprise, mais en utilisant cette fois la méthode d'humidification (Hum). Le mélange pour compression est préparé à partir d'un mélange humide d'excipients sous forme de grains humides mélangés aux Neutres 500/600.

Une partie des excipients forme les grains, l'autre partie des excipients (à savoir ceux qui ne forment pas les grains d'excipients) est ajoutée en phase externe.

Dans un premier temps, on prépare des grains d'excipients présentant la composition centésimale donnée dans le tableau 10.

Une valeur de 3 % d'humidité résiduelle (HR) est ciblée pour les grains d'excipients. La quantité d'eau est ajoutée au fur à et mesure, avec contrôles réguliers de LOD, jusqu'à obtenir la valeur de HR souhaitée (3%).

**Tableau 10**

| **Noms** | **Pourcentages (extraot sec)** | **Pourcentages** |
|---|---|---|
| Mannitol 60 | 71,14 | 69.59 |
| Polyplasdone XL | 17,85 | 17.46 |
| Aspartam | 3,36 | 3.29 |
| L-HPC LH 21 | 6,31 | 6.17 |
| Syloid FP 244 | 1,34 | 1.31 |
| **Eau** | | 2,22 |
| **Total** | 100 | 100 |

On mélange ensuite les grains d'excipients avec les Neutres 500/600 et les excipients supplémentaires, afin d'obtenir le mélange pour compression dont la composition centésimale est donnée dans le tableau 11.

**Tableau 11**

| **Noms** | **Pourcentages** |
|---|---|
| Neutres 500/600 | 20,09 |
| Mélange d'excipients sous forme de grains | 76,00 |
| Syloid FP 244 | 1,95 |
| Pruv® | 1,95 |
| **Total** | 100,00 |

Le tableau 12 ci-dessous représente l'humidité résiduelle mesurée pour le mélange d'excipients humides, sous forme de grains humides, et pour le mélange pour compression dont les compositions sont respectivement données dans les tableaux 11 et 12.

**Tableau 12**

| | **LOD (%)** | **KF (%)** |
|---|---|---|
| Mélange d'excipients sous forme de grains | 3,0 | 2,9 |
| Mélange pour compression | 2,6 | 2,7 |

Pour tous les essais d'humidification, le taux d'HR est mesuré sur les comprimés grâce à la méthode de Karl Fischer.

L'humidité résiduelle est d'environ 3 % pour les comprimés sans séchage (ODT Hum non séchés), et d'environ 1,5 % pour les comprimés séchés pendant 2 heures (ODT Hum séchés).

Le mélange pour compression est comprimé en utilisant le même appareillage que dans l'exemple 1 et à deux forces de compression distincte pour atteindre des valeurs de dureté de 50N ou 70N. La dureté des comprimés est mesurée sur les comprimés sortant de la comprimeuse ou après séchage pendant 1 h ou 2h à 50 °C.

Le tableau 13 ci-dessous donne les caractéristiques des ODT Hum de l'invention, lesdites caractéristiques dépendant de la force de compression exercée (12,5 kN ou 16, 4 kN) et de la durée du séchage (0, 1 ou 2 h).

**Tableau 13**

| **Dureté (N)** | **Force de compression (kN)** | **Séchage (h)** | **Masse (mg)** | **Epaisseur (mm)** | **Friabilité (%)** | **Désag. *in vitro* (s)** | | **Indice de Carr** |
|---|---|---|---|---|---|---|---|---|
| | | | | | | **min** | **max** | |
| 48 | 12,5 | 0 | 450 | 3,26 | 0,69 | 13,6 | 15,8 | 21 |
| 43 | | 1 | 444 | 3,27 | 0,43 | 13,6 | 16,5 | |
| 41 | | 2 | 444 | 3,26 | 0,48 | 14,4 | 16,2 | |
| 73 | 16,4 | 0 | 450 | 3,16 | 0,22 | 13,7 | 17,6 | |
| 59 | | 1 | 443 | 3,16 | 0,13 | 15,8 | 20,1 | |
| 56 | | 2 | 439 | 3,42 | 0,07 | 16,0 | 17,8 | |

Le tableau 14 représente les essais *in vivo* pour des comprimés ODT Hum à 50N.

**Tableau 14 : ODT Hum 50 N**

| **Dureté (N)** | **Temps de séchage (h)** | **Désag. en bouche (s)** | **Effet noyau** | **Sensation en bouche** |
|---|---|---|---|---|
| 50 | 0 | 26 | Pas ou peu | Pas granuleux, agréable |
| | 2 | 24 | faible | Un peu plus pâteux |

Les différents comprimés obtenus par les méthodes de compression directe (ODT CD) et humidification (ODT Hum) sont comparés entre eux et aux comprimés Flashtab® (ODT FT de l'exemple 1) selon leur friabilité, leur temps de désagrégation en bouche, l'effet noyau et la sensation en bouche. Les procédés sont également comparés en termes de force de compression.

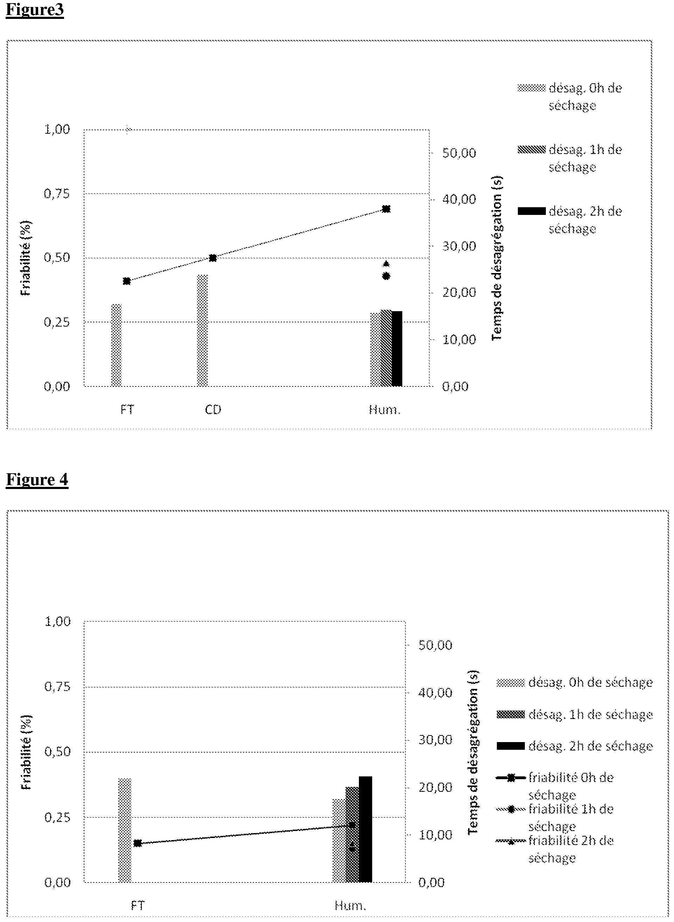

Les résultats obtenus en terme de friabilité et de temps de désagrégation *in vitro,* présentant respectivement une dureté de 50 N et de 70 N sont représentés dans les figures 3 (50 N) et 4 (70 N), au bout de 0, 1 ou 2 h de séchage.

Pour les comprimés de 50 N (figure 3), la friabilité des ODT CD et des ODT Hum sans séchage final (ODT Hum non séchés) est augmentée par rapport celle des ODT FT.

Par contre, la friabilité des ODT Hum après séchage (ODT Hum séchés) reste similaire à celle des ODT FT.

De même, pour les comprimés de 70 N (figure 4), la friabilité des ODT Hum après séchage (ODT Hum séchés) reste similaire à celle des ODT FT.

Concernant le temps de désagrégation *in vitro* des comprimés, il est augmenté par compression directe, alors qu'on note une diminution par humidification, à 50 et 70 N. Cette diminution est moins importante après séchage des comprimés.

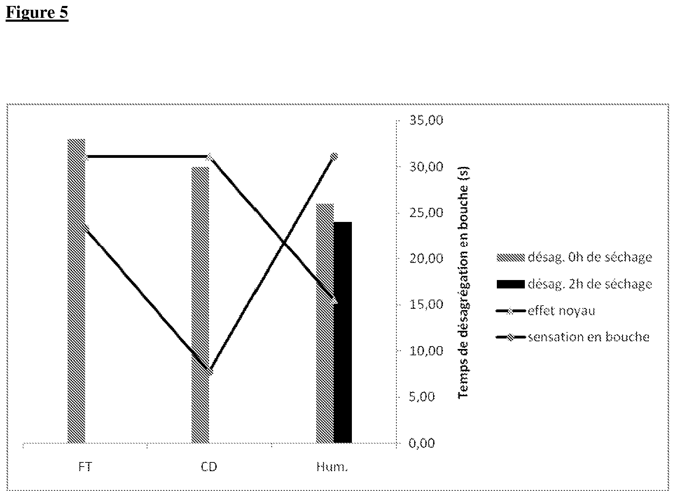

La figure 5 donne les résultats des essais *in vivo* (effet noyau, sensation en bouche et temps de désagrégation *in vivo*) des ODT FT, ODT CD et ODT Hum à 50 N. L'effet noyau et la sensation en bouche étaient peu différents avant et après séchage des comprimés.

Il ressort de ces essais que la méthode de compression moulage par humidification permet avantageusement :
- de diminuer l'effet noyau des comprimés (qu'on cherche à avoir le plus faible possible)
- d'améliorer la sensation en bouche (qu'on cherche à avoir la meilleure possible).

Par ailleurs une diminution assez importante du temps de désagrégation en bouche est également notée pour les comprimés selon l'invention. Les résultats sont assez proches avant et après séchage.

En conclusion, le procédé de l'invention permet de diminuer le temps de désagrégation *in vitro* et *in vivo* des comprimés sans impacter leur friabilité. Cette méthode permet aussi d'améliorer la sensation en bouche et de diminuer l'effet noyau, qui sont des paramètres très importants pour une bonne observance des patients.

### Exemple 3

On prépare des comprimés selon l'invention, en utilisant du Syloid 244FP comme agent d'écoulement à la fois dans les grains d'excipients humides et directement dans le mélange pour compression.

Les tableaux 15 et 16 ci-dessous représentent respectivement la composition centésimale du mélange humide d'excipients, sous forme de grains humides, et celle du mélange pour compression.

**Tableau 15**

| **Noms** | **Pourcentages (extrait sec)** | **Pourcentages** |
|---|---|---|
| Mannitol 60 | 71,14 | 69.59 |
| Polyplasdone XL | 17,85 | 17.46 |
| aspartam | 3,36 | 3.29 |
| L-HPC LH 21 | 6,31 | 6.17 |
| **Syloid 244FP** | 1,34 | 1.31 |
| Eau | | 2,22 |
| **Total** | 100,00 | 100 |

**Tableau 16**

| **Noms** | **Pourcentages** |
|---|---|
| Neutres 500/600 | 20,09 |
| Mélange humide d'excipients (sous forme de grains) | 76,00 |
| **Syloid 244FP** | 1,95 |
| Pruv® | 1,95 |
| **Total** | 100,00 |

Le tableau 17 ci-dessous représente les valeurs de l'humidité résiduelle mesurée pour le mélange humide d'excipients sous forme de grains et pour le mélange pour compression dont les compositions sont respectivement données aux tableaux 15 et 16.

**Tableau 17 :**

| | **LOD (%)** | **KF (%)** |
|---|---|---|
| Mélange d'excipients humides | 3.0 | 2,9 |
| Mélange pour compression | 2.6 | 2.7 |

Le tableau 18 indique les caractéristiques des ODT Hum séchés et non séchés, comprenant du Syloid 244FP en tant qu'agent d'écoulement (voir tab. 15 et 16), en fonction de la force de compression exercée et de la durée de séchage.

**Tableau 18**

| **Dureté (N)** | **Force de compression (kN)** | **Séchage (h)** | **Masse (mg)** | **Epaisseur (mm)** | **Friabilité (%)** | **Désag. *in vitro* (s)** | | **Ecoulement du mélange** |
|---|---|---|---|---|---|---|---|---|
| | | | | | | **min** | **max** | |
| 48 | 12.5 | 0 | 450 | 3,26 | 0,69 | 13.6 | 15.8 | à améliorer |
| 43 | | 1 | 444 | 3,27 | 0,43 | 13.6 | 16.5 | |
| 41 | | 2 | 444 | 3,26 | 0,48 | 14.4 | 16.2 | |
| 73 | 16.4 | 0 | 450 | 3,16 | 0,22 | 13.7 | 17.6 | |
| 59 | | 1 | 443 | 3,16 | 0,13 | 15.8 | 20.1 | |
| 56 | | 2 | 439 | 3,42 | 0,07 | 16,0 | 17.8 | |

### Exemple 4

On a reproduit l'exemple 3, en remplaçant le Syloid 244FP par de l'Aerosil ® 200.

Le tableau 23 représente les essais *in vivo* des ODT Hum ainsi obtenus.

Les tableaux 19 et 20 ci-dessous représentent respectivement la composition centésimale du mélange humide d'excipients se présentant sous la forme de grains et celle du mélange pour compression.

**Tableau 19 : Formule des grains d'excipients**

| **Noms** | **Pourcentages (extrait sec)** | **Pourcentages** |
|---|---|---|
| Mannitol 60 | 71,14 | 69,37 |
| Polyplasdone XL | 17,85 | 17,41 |
| aspartam | 3,36 | 3,28 |
| L-HPC LH 21 | 6,31 | 6,15 |
| **Aerosil ® 200** | 1,34 | 1,31 |
| Eau | | 2,55 |
| **Total** | 100,00 | 100,00 |

**Tableau 20 : Formule du mélange pour compression**

| Noms | Pourcentages |
|---|---|
| Neutre 500/600 | 20.09 |
| Grains d'excipients | 76.00 |
| Aerosil® 200 | 1.95 |
| Pruv® | 1.95 |
| Total | 100.00 |

Le tableau 21 ci-dessous représente les valeurs de l'humidité résiduelle mesurée pour le mélange humide d'excipients, sous forme de grains et pour le mélange pour compression dont les compositions sont respectivement données aux tableaux 19 et 20.

**Tableau 21**

| | **LOD (%)** | **KF (%)** |
|---|---|---|
| Mélange humide d'excipients | 2.9 | 2,9 |
| Mélange pour compression | 3.0 | 2.6 |

Le tableau 22 indique les caractéristiques des ODT Hum séchés et non séchés, comprenant de l'Aerosil ® 200 en tant qu'agent d'écoulement (voir tab. 19 et 20), en fonction de la force de compression exercée et de la durée de séchage.

**Tableau 22**

| **Dureté (N)** | **Force de compression (kN)** | **Séchage (h)** | **Masse (mg)** | **Epaisseur (mm)** | **Friabilité (%)** | **Désag. *in vitro* (s)** | | **Ecoulement du mélange** |
|---|---|---|---|---|---|---|---|---|
| | | | | | | **min** | **max** | |
| 52 | 14.2 | 0 | 454 | 3,35 | 0.63 | 14.5 | 16.6 | à améliorer |
| 36 | | 1 | 442 | 3,37 | 0.49 | 16.9 | 18.8 | |
| 37 | | 2 | 446 | 3,36 | 0.39 | 16.3 | 19.0 | |
| 66 | 17.5 | 0 | 451 | 3,28 | 0.22 | 14.4 | 16.1 | |
| 50 | | 1 | 442 | 3,29 | 0.13 | 17.0 | 18.8 | |
| 47 | | 2 | 339 | 3,28 | 0.13 | 16.7 | 18.8 | |

**Tableau 23 : ODT Hum avec Aerosil ® 200**

| **Dureté (N)** | **Temps de séchage (h)** | **Désag. en bouche (s)** | **Effet noyau** | **Sensation en bouche** |
|---|---|---|---|---|
| 50 | 0 | 28 | Pas ou peu | Assez agréable |
| | 2 | 29 | Pas ou peu | Légèrement pâteux |
| 70 | 0 | 27 | Pas ou peu | Texture agréable |
| | 2 | 24 | Pas ou peu | Texture agréable |

Le remplacement du Syloid® 244 FP par de l'Aerosil® 200 dans les ODT Hum a peu d'influence sur la friabilité, le temps de désagrégation et la sensation en bouche pour des comprimés à 50 N.

A 70 N, les ODT Hum contenant de l'Aerosil® 200 ont un temps de désagrégation plus court et moins d'effet noyau que les ODT Hum contenant du Syloid® 244 FP.

### Exemple 5

Des comprimés selon l'invention sont préparés en utilisant comme solution d'humidification une solution aqueuse de Gelucire® 44/14 à deux concentrations différentes: 5% (m/m) et 15% (m/m). La Gelucire ® 44/14 servant d'agent humectant pour le mélange d'excipients.

### Exemple 5.1 : Solution aqueuse à 5 % de Gelucire® 44/14

Le tableau 20 représente la composition centésimale du mélange humide d'excipients sous forme de grains.

**Tableau 20**

| **Noms** | **Pourcentages en extrait sec** | **Pourcentages** |
|---|---|---|
| Mannitol 60 | 71,06 | 69,29 |
| Polyplasdone XL | 17,83 | 17,39 |
| aspartam | 3,35 | 3,27 |
| L-HPC LH 21 | 6,30 | 6,14 |
| Aerosil® 200 | 1,34 | 1,31 |
| **Solution de Gelucire® 44/14 à 5% m/m** | 0,12 | 2,55 % |
| **Total** | 100,00 | 100,00 |

Le tableau 21 représente la composition du mélange pour compression moulage.

**Tableau 21**

| **Noms** | **Pourcentages** |
|---|---|
| Neutres 500/600 | 20,09 |
| Grains d'excipients | 76,00 |
| **Aerosil® 200** | 1,95 |
| Pruv® | 1,95 |
| **Total** | 100,00 |

Le tableau 22 ci-dessous représente les valeurs de l'humidité résiduelle mesurée pour le mélange d'excipients sous forme de grains et pour le mélange pour compression.

**Tableau 22**

| | **LOD (%)** | **KF (%)** |
|---|---|---|
| Mélange d'excipients sous forme de grains | 2,9 | 3,0 |
| Mélange pour compression | 2,9 | 2,5 |

Le tableau 23 ci-dessous indique les caractéristiques des ODT Hum séchés et non séchés, obtenu par une solution aqueuse de Gelucire® 44/14 à 5% en tant qu'agent humectant, en fonction de la force de compression exercée et de la durée de séchage.

**Tableau 23**

| **Dureté (N)** | **Force de compression (kN)** | **Séchage (h)** | **Masse (mg)** | **Epaisseur (mm)** | **Friabilité (%)** | **Désag. *in vitro* (s)** | | **Ecoulement du mélange** |
|---|---|---|---|---|---|---|---|---|
| | | | | | | **min** | **max** | |
| 51 | 14,0 | 0 | 449 | 3,34 | 0,76 | 14,8 | 16,5 | à améliorer |
| 37 | | 1 | 442 | 3,36 | 0,74 | 15,6 | 17,0 | |
| 34 | | 2 | 441 | 3,36 | 0,77 | 14,8 | 17,6 | |
| 69 | 18,9 | 0 | 446 | 3,24 | 0,19 | 14,7 | 18,3 | |
| 56 | | 1 | 444 | 3,27 | 0,13 | 15,6 | 18,0 | |
| 53 | | 2 | 441 | 3,27 | 0,16 | 16,3 | 19,6 | |

Le tableau 24 ci-dessous représente les essais *in vivo* des ODT Hum comprenant une solution aqueuse de Gelucire® 44/14 à 5% en tant qu'agent humectant.

**Tableau 24**

| **Dureté (N)** | **Temps de séchage (h)** | **Désag. en bouche (s)** | **Effet noyau** | **Sensation en bouche** |
|---|---|---|---|---|
| 50 | 0 | 22 | Pas ou peu | Texture agréable |
| | 2 | 22 | Pas ou peu | Texture agréable |

### Exemple 5.2 : Solution de Gelucire® 44/14 à 15%

Les tableaux 25 et 26 ci-dessous représentent respectivement la composition des grains d'excipients et celle du mélange pour la compression, lorsqu'une solution aqueuse à 15 % de Gelucire® 44/14 est utilisée.

**Tableau 25**

| **Noms** | **Pourcentages en extrait sec** | **Pourcentages** |
|---|---|---|
| Mannitol 60 | 71,06 | 69.21 |
| Polyplasdone XL | 17,83 | 17.36 |
| aspartam | 3,35 | 3.26 |
| L-HPC LH 21 | 6,30 | 6.14 |
| Aerosil® 200 | 1,34 | 1.30 |
| **Solution de Gelucire® 44/14 à 15% m/m** | | 2,68 |
| **Total** | 100,00 | 100,00 |

**Tableau 26**

| **Noms** | **Pourcentages** |
|---|---|
| Neutres 500/600 | 20,09 |
| Grains d'excipients | 76,00 |
| **Aerosil® 200** | 1,95 |
| Pruv® | 1,95 |
| **Total** | 100,00 |

Le tableau 27 ci-dessous représente les valeurs de l'humidité résiduelle mesurée pour le mélange d'excipients, sous forme de grains, et pour le mélange pour compression dont les compositions sont respectivement données aux tableaux 25 et 26.

**Tableau 27**

| | **LOD (%)** | **KF (%)** |
|---|---|---|
| Mélange d'excipients sous forme de grains | 2,9 | 3,1 |
| Mélange pour compression | 2,5 | 2,5 |

Le tableau 28 ci-dessous indique les caractéristiques des ODT Hum séchés et non séchés, obtenu par une solution de Gelucire® 44/14 à 15% en tant qu'agent humectant, en fonction de la force de compression exercée et de la durée de séchage.

**Tableau 28**

| **Dureté (N)** | **Force de compression (kN)** | **Séchage (h)** | **Masse (mg)** | **Epaisseur (mm)** | **Friabilité (%)** | **Désag. *in vitro* (s)** | |
|---|---|---|---|---|---|---|---|
| | | | | | | **min** | **max** |
| 50 | 14,8 | 0 | 454 | 3,37 | 0,82 | 15,0 | 17,0 |
| 36 | | 1 | 449 | 3,37 | 0,73 | 17,2 | 18,9 |
| 34 | | 2 | 447 | 3,35 | 0,81 | 15,7 | 18,5 |
| 74 | 20,3 | 0 | 456 | 3,29 | 0,23 | 15,6 | 17,4 |
| 54 | | 1 | 448 | 3,31 | 0,18 | 16,8 | 18,0 |
| 52 | | 2 | 447 | 3,28 | 0,22 | 17,5 | 18,9 |

Le tableau 29 ci-dessous représente les essais *in vivo* des ODT Hum comprenant une solution de Gelucire® 44/14 à 15% en tant qu'agent humectant.

**Tableau 29**

| **Dureté (N)** | **Temps de séchage (h)** | **Désag. en bouche (s)** | **Effet noyau** | **Sensation en bouche** |
|---|---|---|---|---|
| 50 | 0 | 21 | Pas ou peu | Texture agréable |
| | 2 | 24 | Pas ou peu | Texture agréable |

Il n'y a pas de différence notable entre les ODT Hum comprenant une solution de Gelucire® 44/14 à 5% ou à 15%, en termes de friabilité et temps de désagrégation.

Les temps de désagrégation des ODT Hum sont similaires à ceux des ODT FT pour les ODT Hum à 50 N, et légèrement inférieurs pour les ODT Hum à 70 N.

Concernant les essais *in vivo,* l'emploi de Gelucire® 44/14 permet de diminuer l'effet noyau en bouche. La texture même du comprimé est appréciée.

## Revendications

1. Procédé de compression moulage (« compression molding ») pour la préparation d'un comprimé orodispersible présentant une dureté allant de 30 à 80 N, et de préférence de 40 à 75 N, une friabilité inférieure à 1 % et de préférence inférieure à 0,5 %, une désintégration en bouche inférieure à 60 secondes et de préférence inférieure à 40 secondes, **caractérisé en ce que** ledit procédé comprend les étapes suivantes :
- préparation d'un mélange d'excipients, ledit mélange d'excipients se trouvant sous forme de grains, ledit mélange d'excipients étant choisi dans le groupe comprenant un diluant, un désintégrant, un édulcorant, un liant, un agent d'écoulement, un humectant ou agent mouillant, un lubrifiant, un agent aromatisant, un colorant et leurs mélanges,
- humidification dudit mélange d'excipients à l'aide d'une solution d'humidification en une quantité permettant d'obtenir un mélange humide d'excipients présentant une humidité résiduelle ou teneur en eau allant de 2 à 4%,
- préparation de microcristaux ou de microgranules de principe actif enrobés,
- mélange des microcristaux ou microgranules de principe actif enrobés d'une part et du mélange humide d'excipients sous forme de grains d'autre part, afin d'obtenir un mélange humide pour compression présentant une humidité résiduelle ou teneur en eau allant de 2 à 3 %,
- éventuellement, ajout au mélange humide pour compression, tel que préparé ci-dessus, d'excipients choisis dans le groupe comprenant un agent d'écoulement, un lubrifiant, un arôme, un édulcorant et leurs mélanges,
- compression du mélange pour compression préparé ci-dessus pour obtenir un comprimé,
- éventuellement séchage du comprimé ainsi obtenu.

2. Procédé selon la revendication 1, **caractérisé en ce que** le mélange d'excipients comprend au moins un humectant choisi dans le groupe comprenant les poloxamers, de préférence le « poloxamer 188 » ou le « poloxamer 407 », les macrogols, les macrogolglycérides, les polysorbates, ledit humectant étant de préférence un macrogolglycéride tel que le stéaroyl macrogol-32 glycéride ou le lauroyl macrogol-32 glycéride commercialisé sous la dénomination Gelucire ® 44/14.

3. Procédé selon la revendication 1 ou 2, **caractérisé en ce que** l'humidification du mélange d'excipients sous forme de grains est effectuée à l'aide d'une solution d'humidification choisie dans le groupe comprenant de l'eau, une solution aqueuse d'humidification, un humectant, une solution alcoolique et leurs mélanges.

4. Procédé selon la revendication 3, **caractérisé en ce que** la solution d'humidification comprend une solution aqueuse d'humidification et un humectant, ledit humectant étant de préférence le lauroyl macrogol-32 glycéride commercialisé sous la dénomination Gelucire ® 44/14.

5. Procédé selon l'une quelconque des revendications 1 à 4, **caractérisé en ce que** le mélange d'excipients sous forme de grains comprend :
- de 65 à 90 %, et de préférence de 70 à 80 %, d'un diluant choisi dans le groupe comprenant le mannitol, le xylitol, le sorbitol, le maltitol et leurs mélanges, ledit diluant étant de préférence le mannitol commercialisé sous la dénomination Mannitol 60,
- de 2 à 25 %, et de préférence de 10 à 20 %, d'un désintégrant choisi dans le groupe comprenant la crospovidone, la croscarmellose sodique (AcDiSol®), le carboxyméthylamidon sodique (Explotab ®) et leurs mélanges, ledit désintégrant étant de préférence la crospovidone commercialisée sous la dénomination Polyplasdone ® XL,
- de 1 à 8 %, et de préférence de 3 à 5 %, d'un édulcorant choisi dans le groupe comprenant l'aspartam, l'acesulfame de potassium, la saccharinate de sodium, le sucralose et leurs mélanges, ledit édulcorant étant de préférence l'aspartam,
- de 3 à 10 %, et de préférence de 5 à 8 %, d'un liant choisi dans le groupe comprenant l'hydroxypropylcellulose faiblement substituée, la gomme arabique, l'amidon de maïs, l'amidon prégélatinisé, les maltodextrines et leurs mélanges, ledit liant étant de préférence la gomme arabique et/ou l'hydroxypropylcellulose commercialisée sous la dénomination L-HPC LH 21,
- de 0 à 5 %, et de préférence de 1 à 3 %, d'un agent d'écoulement choisi dans le groupe comprenant de la silice, de préférence celle commercialisée sous la dénomination Syloid ® 244 FP, de la silice colloïdale hydrophobe, de préférence celle commercialisée sous la dénomination Aerosil ® R 972, de la silice précipitée, de préférence celle commercialisée sous la dénomination Aerosil ® 200, et leurs mélanges,
- de 0 à 5 %, et de préférence de 0,1 à 3 %, d'un humectant, ledit humectant étant tel que défini à la revendication 2,
- de 0 % à 5 % d'un lubrifiant, ledit lubrifiant étant de préférence un lubrifiant hydrophile choisi dans le groupe comprenant le stéaryl fumarate de sodium, le lauryl sulfate de sodium, ledit lubrifiant hydrophile étant de préférence le stéaryl fumarate de sodium commercialisé par exemple sous la dénomination Pruv ®,
- de 0 à 8 %, et de préférence de 0,5 à 4 %, d'un agent aromatisant et/ou d'un colorant,
- de l'eau qsp 100 %,
les pourcentages étant des pourcentages en poids par rapport au poids total du mélange d'excipients sous forme de grains.

6. Procédé selon l'une quelconque des revendications 1 à 5, **caractérisé en ce que** les excipients qui ne se trouvent pas dans le mélange humide d'excipients sous forme de grains, à savoir ceux pouvant être ajoutés, en plus du mélange humide d'excipients, dans le mélange pour compression, sont choisis dans le groupe comprenant :
- de 1 à 5 %, de préférence de 2 à 4 %, d'un agent d'écoulement,
- de 1 % à 5 %, de préférence de 2 à 4 %, d'un lubrifiant,
- de 0 à 5 %, et de préférence de 0,5 à 4 %, d'un agent aromatisant et/ou d'un colorant,
lesdits agents d'écoulement et lubrifiant étant tels que définis à la revendication 5,
les pourcentages étant des pourcentages en poids par rapport au poids total du mélange pour compression.

7. Procédé selon l'une quelconque des revendications 1 à 6, **caractérisé en ce que** le mélange pour compression comprend :
- de 5 à 40 %, de préférence de 10 à 30 %, et plus préférentiellement encore de 15 à 25 % de microcristaux ou de microgranules de principe actif enrobés,
- de 55 à 95 %, de préférence de 65 à 85 %, et plus préférentiellement encore de 70 à 80 % du mélange humide d'excipients sous forme de grains,
- de 0 à 10 %, de préférence de 1 à 7 %, et plus préférentiellement encore de 2 à 5 % d'excipients ne se trouvant pas dans le mélange humide d'excipients sous forme de grains,
les pourcentages étant des pourcentages en poids par rapport au poids total du mélange pour compression.

8. Procédé selon l'une quelconque des revendications 1 à 7, **caractérisé en ce que caractérisé en ce que** le mélange pour compression est comprimé à l'aide de forces de compression allant de 8 à 22 kN (kNewtons), de préférence de 10 à 20 kN, et plus préférentiellement encore de 12 kN à 18 kN.

9. Procédé selon l'une quelconque des revendications 1 à 8, **caractérisé en ce que** le séchage du comprimé obtenu à l'issue de l'étape de compression, est effectué à une température allant de 35 à 65 °C, de préférence de 45 à 55 °C, pendant un temps allant de 30 min à 3 heures, et de préférence de 1 à 2 heures.

10. Comprimé tel qu'obtenu selon le procédé de l'une quelconque des revendications 1 à 9, **caractérisé en ce qu'**il présente :
- pas ou peu d'effet noyau en bouche, et/ou
- une texture agréable en bouche et/ou un goût agréable en bouche.

## Patentansprüche

1. Formpressverfahren ("compression molding") zur Herstellung einer Schmelztablette mit einer Härte von 30 bis 80 N, vorzugsweise von 40 bis 75 N, einer Sprödigkeit von weniger als 1%, vorzugsweise weniger als 0,5%, einer Zersetzung im Mund in weniger als 60 Sekunden, vorzugsweise weniger als 40 Sekunden, **dadurch gekennzeichnet, dass** das Verfahren die folgenden Schritte umfasst:
- Herstellen eines Hilfsstoffgemischs, wobei das Hilfsstoffgemisch in Körnchenform vorliegt, wobei das Hilfsstoffgemisch ausgewählt ist aus der Gruppe, umfassend ein Verdünnungsmittel, ein Zerfallsmittel, ein Süßungsmittel, ein Bindemittel, ein Fließmittel, ein Feuchthaltemittel oder Benetzungsmittel, ein Schmiermittel, einen Aromastoff, einen Farbstoff und Mischungen davon,
- Befeuchten des Hilfsstoffgemischs unter Verwendung einer Befeuchtungslösung in einer Menge, dies es ermöglicht ein feuchtes Hilfsstoffgemischs mit einer Restfeuchtigkeit oder einem Wassergehalt im Bereich von 2 bis 4% zu erhalten,
- Herstellen von Mikrokristallen oder Mikrogranulaten eines beschichteten Wirkstoffs,
- Mischen der Mikrokristalle oder Mikrogranulate des beschichteten Wirkstoffs einerseits und des feuchten Hilfsstoffgemischs, das in Körnchenform vorliegt, andererseits, um ein feuchtes Gemisch für die Pressung mit einer Restfeuchtigkeit oder einem Wassergehalt im Bereich von 2 bis 3% zu erhalten,
- gegebenenfalls Zugeben von Hilfsstoffen, ausgewählt aus der Gruppe, umfassend ein Fließmittel, ein Schmiermittel, einen Aromastoff, ein Süßungsmittel und Mischungen davon, zu dem wie oben hergestellten feuchten Gemisch für die Pressung,
- Pressen des wie oben hergestellten Gemischs für die Pressung, um eine Tablette zu erhalten,
- gegebenenfalls Trocknen der so erhaltenen Tablette.

2. Verfahren gemäß Anspruch 1, **dadurch gekennzeichnet, dass** das Hilfsstoffgemisch mindestens ein Feuchthaltemittel umfasst, ausgewählt aus der Gruppe, umfassend Poloxamere, vorzugsweise "Poloxamer 188" oder "Poloxamer 407", Makrogole, Makrogolglyceride, Polysorbate, wobei das Feuchthaltemittel vorzugsweise ein Makrogolglycerid wie Stearoylmakrogol-32-glycerid oder Lauroylmakrogol-32-glycerid, das unter der Bezeichnung Gelucire ® 44/14 vertrieben wird, ist.

3. Verfahren gemäß Anspruch 1 oder 2, **dadurch gekennzeichnet, dass** das Befeuchten des Hilfsstoffgemischs, das in Körnchenform vorliegt, unter Verwendung einer Befeuchtungslösung durchgeführt wird, ausgewählt aus der Gruppe, umfassend Wasser, eine wässrige Befeuchtungslösung, ein Feuchthaltemittel, eine alkoholische Lösung und Mischungen davon.

4. Verfahren gemäß Anspruch 3, **dadurch gekennzeichnet, dass** die Befeuchtungslösung eine wässrige Befeuchtungslösung und ein Feuchthaltemittel umfasst, wobei das Feuchthaltemittel vorzugsweise Lauroyl-Macrogol-32-Glycerid ist, das unter der Bezeichnung Gelucire ® 44/14 vertrieben wird.

5. Verfahren gemäß einem der Ansprüche 1 bis 4, **dadurch gekennzeichnet, dass** das Hilfsstoffgemisch, das in Körnchenform vorliegt, umfasst:
- 65 bis 90%, vorzugsweise 70 bis 80%, eines Verdünnungsmittels, ausgewählt aus der Gruppe, umfassend Mannit, Xylit, Sorbit, Maltit und Mischungen davon, wobei das Verdünnungsmittel vorzugsweise Mannit ist, das unter der Bezeichnung Mannitol 60 vertrieben wird,
- 2 bis 25%, vorzugsweise 10 bis 20%, eines Zerfallsmittels, ausgewählt aus der Gruppe, umfassend Crospovidon, Croscarmellose-Natrium (AcDiSol ®), Natriumcarboxymethylstärke (Explotab ®) und Mischungen davon, wobei das Zerfallsmittel vorzugsweise Crospovidon ist, das unter der Bezeichnung Polyplasdone ® XL vertrieben wird,
- 1 bis 8%, vorzugsweise 3 bis 5%, eines Süßungsmittel, ausgewählt aus der Gruppe, umfassend Aspartam, Kaliumacesulfam, Natriumsaccharinat, Sucralose und Mischungen davon, wobei das Süßungsmittel vorzugsweise Aspartam ist,
- 3 bis 10%, vorzugsweise 5 bis 8%, eines Bindemittels, ausgewählt aus der Gruppe, umfassend schwach substituierte Hydroxypropylcellulose, Gummi arabicum, Maisstärke, vorgelatinierte Stärke, Maltodextrine und Mischungen davon, wobei das Bindemittel vorzugsweise Gummi arabicum und/oder Hydroxypropylcellulose ist, die unter der Bezeichnung L-HPC LH 21 vertrieben wird,
- 0 bis 5%, vorzugsweise 1 bis 3%, eines Fließmittels, ausgewählt aus der Gruppe, umfassend Siliciumdioxid, vorzugsweise jenes, das unter der Bezeichnung Syloid ® 244 FP vertrieben wird, hydrophobes kolloidales Siliciumdioxid, vorzugsweise, jenes das unter der Bezeichnung Aerosil ® R 972 vertrieben wird, gefälltes Siliciumdioxid, vorzugsweise, jenes, das unter der Bezeichnung Aerosil ® 200 vertrieben wird, und Mischungen davon,
- 0 bis 5%, vorzugsweise 0,1 bis 3%, eines Feuchthaltemittels, wobei das Feuchthaltemittel wie in Anspruch 2 definiert ist.
- von 0% bis 5% eines Schmiermittels, wobei das Schmiermittel vorzugsweise ein hydrophiles Schmiermittel ist, ausgewählt aus der Gruppe, umfassend Natriumstearylfumarat, Natriumlaurylsulfat, wobei das hydrophile Schmiermittel vorzugsweise Natriumstearylfumarat ist, das unter der Bezeichnung Pruv ® vertrieben wird,
- 0 bis 8%, vorzugsweise 0,5 bis 4%, eines Aromastoffs und/oder eines Farbstoffs,
- Wasser *qs* für 100%,
wobei die Prozentangaben Gewichtsprozente sind, bezogen auf das Gesamtgewicht des Hilfsstoffgemischs, das in Körnchenform vorliegt.

6. Verfahren gemäß einem der Ansprüche 1 bis 5, **dadurch gekennzeichnet, dass** die Hilfsstoffe, die nicht im feuchten Hilfsstoffgemisch, das in Körnchenform vorliegt, enthalten sind, das heißt, diejenigen, die zusätzlich zu dem feuchten Hilfsstoffgemisch dem Gemisch für die Pressung hinzugefügt werden können, ausgewählt sind aus der Gruppe, umfassend:
- 1 bis 5%, vorzugsweise 2 bis 4%, eines Fließmittels,
- 1 bis 5%, vorzugsweise 2% bis 4%, eines Schmiermittels,
- 0 bis 5%, vorzugsweise 0,5 bis 4%, eines Aromastoffs und/oder eines Farbstoffs,
wobei das Fließmittel und das Schmiermittel wie in Anspruch 5 definiert sind, wobei die Prozentangaben Gewichtsprozente sind, bezogen auf das Gesamtgewicht des Gemischs für die Pressung.

7. Verfahren gemäß einem der Ansprüche 1 bis 6, **dadurch gekennzeichnet, dass** das Gemisch für die Pressung umfasst:
- 5 bis 40%, vorzugsweise 10 bis 30%, und besonders bevorzugt 15 bis 25%, Mikrokristalle oder Mikropartikel des beschichteten Wirkstoffs,
- 55 bis 95%, vorzugsweise 65 bis 85%, und besonders bevorzugt 70 bis 80%, des feuchten Hilfsstoffgemischs, das in Körnchenform vorliegt,
- 0 bis 10%, vorzugsweise 1 bis 7% und besonders bevorzugt 2 bis 5% der Hilfsstoffe, die in dem feuchten Hilfsstoffgemisch, das in Körnchenform vorliegt, nicht enthalten sind,
wobei die Prozentangaben Gewichtsprozente sind, bezogen auf das Gesamtgewicht des Gemischs für die Pressung.

8. Verfahren gemäß einem der Ansprüche 1 bis 7, **dadurch gekennzeichnet, dass** das Gemisch für die Pressung mittels Kompressionskräften im Bereich von 8 bis 22 kN (kNewton), vorzugsweise 10 bis 20 kN, besonders bevorzugt 12 kN bis 18 kN, gepresst wird.

9. Verfahren gemäß einem der Ansprüche 1 bis 8, **dadurch gekennzeichnet, dass** das Trocknen der am Ende des Pressschrittes erhaltenen Tablette bei einer Temperatur im Bereich von 35 bis 65°C, vorzugsweise 45 bis 55°C, über einen Zeitraum von 30 Minuten bis 3 Stunden, vorzugsweise 1 bis 2 Stunden, erfolgt.

10. Tablette, erhalten gemäß dem Verfahren gemäß einem der Ansprüche 1 bis 9, **dadurch gekennzeichnet, dass** sie:
- keinen oder einen geringen körnigen Effekt im Mund aufweist und/oder
- eine angenehme Textur im Mund und/oder einen angenehmen Geschmack im Mund aufweist.

## Claims

1. A compression molding process for preparing an orodispersible tablet having a hardness ranging from 30 to 80 N, preferably from 40 to 75 N, a friability of less than 1% and preferably less than 0.5%, a disintegration in the mouth of less than 60 seconds and preferably less than 40 seconds, **characterized in that** said process comprises the following steps:
- preparation of a mixture of excipients, said mixture of excipients being in the form of grains, said mixture of excipients being chosen from the group comprising a diluent, a disintegrant, a sweetener, a binder, a flow agent, a humectant or wetting agent, a lubricant, a flavoring agent, a colorant and mixtures thereof,
- wetting said mixture of excipients by using a wetting solution in an amount making it possible to obtain a wet mixture of excipients having a residual humidity or water content ranging from 2% to 4%,
- preparation of coated microcrystals or microgranules of active principle,
- mixing of the coated microcrystals or microgranules of active principle, on the one hand, and of the wet mixture of excipients in the form of grains, on the other hand, in order to obtain a wet mixture for compression having a residual humidity or water content ranging from 2% to 3%,
- optionally, adding to the wet mixture for compression, as prepared above, of excipients chosen from the group comprising a flow agent, a lubricant, a flavoring, a sweetener and mixtures thereof,
- compression of the mixture for compression prepared above to obtain a tablet,
- optionally, drying of the tablet thus obtained.

2. The process as claimed in claim 1, **characterized in that** the mixture of excipients comprises at least one humectant chosen from the group comprising poloxamers, preferably "poloxamer 188" or "poloxamer 407", macrogols, macrogol glycerides, polysorbates, said humectant preferably being a macrogol glyceride such as stearoyl macrogol-32 glyceride or lauroyl macrogol-32 glyceride sold under the name Gelucire® 44/14.

3. The process as claimed in claim 1 or 2, **characterized in that** the wetting of the mixture of excipients in the form of grains is performed using a wetting solution chosen from the group comprising water, an aqueous wetting solution, a humectant, an alcoholic solution and mixtures thereof.

4. The process as claimed in claim 3, **characterized in that** the wetting solution comprises an aqueous wetting solution and a humectant, said humectant preferably being lauroyl macrogol-32 glyceride sold under the name Gelucire ® 44/14.

5. The process as claimed in any one of claims 1 to 4, **characterized in that** the mixture of excipients in the form of grains comprises:
- from 65% to 90% and preferably from 70% to 80%, of a diluent chosen from the group comprising mannitol, xylitol, sorbitol, maltitol and mixtures thereof, said diluent preferably being mannitol sold under the name Mannitol 60,
- from 2% to 25% and preferably from 10% to 20%, of a disintegrant chosen from the group comprising crospovidone, sodium croscarmellose (AcDiSol®), sodium carboxymethyl starch (Explotab ®) and mixtures thereof, said disintegrant preferably being crospovidone sold under the name Polyplasdone ® XL,
- from 1% to 8% and preferably from 3% to 5% of a sweetener chosen from the group comprising aspartame, potassium acesulfame, sodium saccharinate, sucralose and mixtures thereof, said sweetener preferably being aspartame,
- from 3% to 10% and preferably from 5% to 8%, of a binder chosen from the group comprising weakly substituted hydroxypropylcellulose, gum arabic, corn starch, pregelatinized starch, maltodextrins and mixtures thereof, said binder preferably being gum arabic and/or hydroxypropylcellulose sold under the name L-HPC LH 21,
- from 0% to 5% and preferably from 1% to 3% of a flow agent chosen from the group comprising silica, preferably that sold under the name Syloid ® 244 FP, hydrophobic colloidal silica, preferably that sold under the name Aerosil ® R 972, precipitated silica, preferably that sold under the name Aerosil ® 200, and mixtures thereof,
- from 0 to 5% and preferably from 0.1% to 3% of a humectant, said humectant being as defined in claim 2,
- from 0 to 5% of a lubricant, said lubricant preferably being a hydrophilic lubricant chosen from the group comprising sodium stearyl fumarate, sodium lauryl sulfate, said hydrophilic lubricant preferably being sodium stearyl fumarate sold, for example, under the name Pruv ®,
- from 0 to 8% and preferably from 0.5% to 4% of a flavoring agent and/or colorant,
- water qs 100%,
the percentages being weight percentages relative to the total weight of the mixture of excipients in the form of grains.

6. The process as claimed in any one of claims 1 to 5, **characterized in that** the excipients that are not in the wet mixture of excipients in the form of grains, namely those that may be added in addition to the wet mixture of excipients, in the mixture for compression, are chosen from the group comprising:
- from 1% to 5%, preferably from 2% to 4%, of a flow agent,
- from 1% to 5%, preferably from 2% to 4%, of a lubricant,
- from 0 to 5% and preferably from 0.5% to 4% of a flavoring agent and/or a colorant,
said flow agents and lubricant being as defined in claim 5,
the percentages being weight percentages relative to the total weight of the mixture for compression.

7. The process as claimed in any one of claims 1 to 6, **characterized in that** the mixture for compression comprises:
- from 5% to 40%, preferably from 10% to 30% and even more preferentially from 15% to 25% of coated microcrystals or microgranules of active principle,
- from 55% to 95%, preferably from 65% to 85% and even more preferentially from 70% to 80% of the wet mixture of excipients, preferably in the form of grains,
- from 0 to 10%, preferably from 1% to 7% and even more preferentially from 2% to 5% of excipients that are not present in the wet mixture of excipients in the form of grains,
the percentages being weight percentages relative to the total weight of the mixture for compression.

8. The process as claimed in any one of claims 1 to 7, **characterized in that** the mixture for compression is compressed using compression forces ranging from 8 to 22 kN (kNewtons), preferably from 10 to 20 kN and even more preferentially from 12 kN to 18 kN.

9. The process as claimed in any one of claims 1 to 8, **characterized in that** the drying of the tablet obtained after the compression step is performed at a temperature ranging from 35 to 65°C, preferably from 45 to 55°C, for a time ranging from 30 minutes to 3 hours and preferably from 1 to 2 hours.

10. The tablet such as obtained according to the process of any one of claims 1 to 9, **characterized in that** it has:
- little or no core effect in the mouth, and/or
- a pleasant texture in the mouth and/or a pleasant taste in the mouth.
